# EUROPÄISCHE PATENTSCHRIFT

(11) **EP 0 831 061 B1**
(45) Veröffentlichungstag und Bekanntmachung des Hinweises auf die Patenterteilung: **26.05.1999**
(21) Anmeldenummer: 97115804.3
(22) Anmeldetag: 11.09.1997
(51) Int. Cl.: C02F 1/20

(54) **Rückgewinnung von Alkoholen aus Prozessabwässern der Siliconharzherstellung**
Recovery of alcohol from silicone resin manufacturing waste water
Procédé de récupération d'alcool à partir des eaux usées provenant de la fabrication de résine silicone

(30) Priorität: 18.09.1996 DE 19638123
(43) Veröffentlichungstag der Anmeldung: 25.03.1998
(73) Patentinhaber: Wacker-Chemie GmbH, 81737 München (DE)
(72) Erfinder: Mautner, Konrad, Dr., 84489 Burghausen (DE); Schuhbeck, Erwin, 84489 Burghausen (DE)
(74) Vertreter: Fritz, Helmut, Dr.

(56) Entgegenhaltungen:
- EP-A- 0 675 128
- DD-A- 228 550
- US-A- 3 615 402
- US-A- 5 198 518
- US-A- 5 378 788

## Beschreibung

Die Erindung betrifft ein Verfahren zur Aufarbeitung des Alkohol enthaltenden Abwassers, welches bei der Hydrolyse von alkoxylierten und teilalkoxylierten Kohlenwasserstoffchlorsilanen anfällt. Der Alkohol wird dabei abgetrennt.

Siliconharze bestehen aus räumlichen Netzwerken, die aus tri- oder tetrafunktionellen Siliciumeinheiten R¹SiO_{3/2} oder SiO_{4/2} gebildet werden, wobei R¹ ausgewählt ist aus aliphatischen oder aromatischen Kohlenwasserstoffresten. Daneben können noch lineare Einheiten R¹₂SiO und/oder monofunktionelle Einheiten R¹₃SiO_{1/2} vorhanden sein. Die Hydrolyse von trifunktionellen Chlorsilanen oder Siliciumtetrachlorid mit Wasser führt zu hohem Gelanteil im Hydrolysat, auch wenn in Gegenwart von organischen Lösungsmitteln, wie Toluol und Aceton gearbeitet wird.

Deshalb wurde sehr früh die in der DE-A-873 433 beschriebene Harzherstellung aus teilalkoxylierten Chlorsilanen oder Alkoxysilanen eingeführt, bei der die Bildung von Gelanteilen stark herabgesetzt ist. Nach der Hydrolyse fällt neben dem Harzprodukt eine alkoholhaltige Wasserphase an, die neben der aus den Chlorsilanen entstandenen Salzsäure auch Siliconharzreste und Gelanteile enthält.

Um die Harzherstellung wirtschaftlich zu gestalten und zur Abfallvermeidung, ist es von großem Interesse, den eingesetzten Alkohol aus der wäßrigen Phase möglichst vollständig und einfach wiederzugewinnen. Das größte Problem bei der Aufarbeitung dieses Prozeßstromes sind die Harz- und Gelanteile. Wird der Alkohol in einer Destillationsblase oder Destillationskolonne aus dem Gemisch herausdestilliert, so treten an den Wärmetauschern nach kurzer Zeit Beläge durch aufwachsendes Siliconharz auf, die den Wärmeübergang behindern und die periodisch abgereinigt werden müssen. Auch bei direktem Einblasen von Wasserdampf bilden sich im Sauren aus den Gelanteilen Beläge an den Wandungen.

Ein weiteres Problem bei der Aufarbeitung der wäßrig-alkoholischen Phase ist in vielen Fällen die Säure, die als Kondensationskatalysator notwendig ist. Da häufig Salzsäure, die sich bei der Hydrolyse der teilalkoxylierten Silane bildet, verwendet wird, entstehen leichter flüchtige Alkylhalogenide beim Erhitzen aus den Alkoholen und aus HCl, die wiederum Emissionsprobleme verursachen.

Die Dampfstrippung von organisch belasteten Abwässern wird eingesetzt, um flüchtige organische Bestandteile zu entfernen. Als Kriterium für die erfolgreiche Durchführung der Dampfstrippung von Abwässern sind in Hwang, Y.-L. et al.; Ind. Eng. Chem. Res. **1992**, 31, 1753-1759 und Hwang, Y.-L. et al.; Ind. Eng. Chem. Res. **1992**, 31, 1759- 1768 eine Dampf-Flüssig-Gleichgewichtskonstante bei unendlicher Verdünnung K^{∞} > 1 gefordert. Alkohole weisen niedrige K^{∞}-Werte auf, z.B. Ethanol -0,4 und Methanol -0,5. In Hassan, S.Q.; J. Air Waste Manage. Assoc., **1992**, 42, 936-943 ist als Kriterium für die erfolgreiche Durchführung der Dampfstrippung eine Löslichkeit des organischen Bestandteils in Wasser von höchstens 1000 ppm angegeben. Dampfstrippung ist demnach völlig ungeeignet, um Alkohole wie Ethanol oder Methanol aus einer wäßrigen Lösung zu entfernen.

In der DD-A-228 550 ist die Rückgewinnung von Alkohol aus der wäßrig-alkoholischen Salzsäure, die bei der Herstellung von Methylsiliconharzen anfällt, durch Einleiten von Wasserdampf beschrieben. Es wird darin aber nicht beschrieben, wie die Durchführung geschieht und wie die im salzsauren Medium auftretenden Gelablagerungen und die Alkylchloridemissionen vermieden werden können.

In der US-A5 378 788 ist die Herstellung von linearen Polyorganosiloxanen durch Dialkoxysilanen und Salzsäure beschrieben. Die Reaktion wird durch Zugabe von Metalloxid beendet, wobei der pH-Wert der Reaktionsmischung auf einen Wert bis zu 9 heraufgesetzt wird. Danach werden nicht reagiertes Ausgangsmaterial, Wasser und Alkohol abgetrennt.

Aufgabe der Erfindung war, den Alkohol aus Prozeßabwässern der Siliconharzherstellung zurückzugewinnen, die Emissionen von Alkylhalogeniden drastisch zu senken und die Belagbildung auf Apparateteilen zu unterbinden.

Die Erfindung betrifft ein Verfahren zur Aufarbeitung des Alkohol enthaltenden Abwassers, welches bei der Hydrolyse von Silanen der allgemeinen Formel I

R¹ₐSiCl_{b}OR²_{4-a-b} (I),

in der
- **R**^{**1**}: Wasserstoffatome oder gleiche oder verschiedene einwertige, gegebenenfalls halogensubstituierte, SiC-gebundene C₁-C₁₈-Kohlenwasserstoffreste,
- **R**^{**2**}: gleiche oder verschiedene einwertige C₁-C₁₀-Kohlenwasserstoffreste,
- **a**: 0, 1, 2 oder 3, durchschnittlich 0,3 bis 1,9 und
- **b**: 0, 1, 2, 3 oder 4, durchschnittlich 0,0 bis 3,0
bedeuten, mit der Maßgabe, daß die Summe **a+b** durchschnitlich höchstens 3,5 beträgt,
zur Herstellung von Siliconharzen anfällt, bei dem in einem ersten Schritt das Abwasser mit Lauge alkalisch gestellt wird und
in einem zweiten Schritt aus der entstandenen alkalischen Lösung oder Suspension der Alkohol durch Einblasen von Wasserdampf entfernt wird.

Beispiele für Reste **R**^{**1**} sind Alkylreste, wie der Methyl-, Ethyl-, n- Propyl-, iso-Propyl-, n-Butyl-, iso-Butyl-, tert.-Butyl-, n-Pentyl-, iso-Pentyl-, neo-Pentyl-, tert.-Pentylrest, Hexylreste, wie der n-Hexylrest, Heptylreste, wie der n-Heptylrest, Octylreste, wie der n-Octylrest und iso-Octylreste, wie der 2,2,4-Trimethylpentylrest, Nonylreste, wie der n-Nonylrest, Decylreste, wie der n-Decylrest, Dodecylreste, wie der n-Dodecylrest, Octadecylreste, wie der n-Octadecylrest; Alkenylreste, wie der Vinyl- und der Allylrest; Cycloalkylreste, wie Cyclopentyl-, Cyclohexyl-, Cycloheptylreste und Methylcyclohexylreste; Arylreste, wie der Phenyl- Naphthyl- und Anthryl-und Phenanthrylrest; Alkarylreste, wie o-, m-, p-Tolylreste, Xylylreste und Ethylphenylreste; Aralkylreste, wie der Benzylrest, der alpha- und der β-Phenylethylrest.

Beispiele für halogensubstituierte Reste **R**^{**1**} sind Halogenalkylreste, wie der 3,3,3-Trifluor-n-propylrest, der 2,2,2,2',2',2'-Hexafluorisopropylrest, der Heptafluorisopropylrest, und Halogenarylreste, wie der o-, m-, und p-Chlorphenylrest.

Besonders bevorzugt als Reste **R**^{**1**} sind die nicht substituierten C₁-C₆-Kohlenwasserstoffreste, insbesondere der Methyl- und Phenylrest.

Beispiele für den Rest **R**^{**2**} mit Kohlenwasserstoffresten mit 1 - 10 C-Atomen sind vorstehend bei den Beispielen für **R**^{**1**} aufgeführt. Vorzugsweise bedeutet der Rest **R**^{**2**} lineare und verzweigte C₁-C₄-Kohlenwasserstoffreste, insbesondere den Methyl- und Ethylrest.

Der Alkoholgehalt des Alkohol enthaltenden Abwassers der Hydrolyse (Prozeßabwasser) kann stark variieren und bewegt sich in der Regel bei 5 bis 50 Gew.-%. Im bevorzugten Fall für den Ethanol liegt der Gehalt bei 15 bis 45 Gew.-%.

Die Silane der allgemeinen Formel I können teil- oder vollalkoxyliert sein, d.h. sie können Chloratome enthalten. Ein Anteil von vorzugsweise höchstens 10 Gew.-%, insbesondere höchstens 2 Gew.-%, der Silane der allgemeinen Formel I, kann lediglich Chloratome, aber keine Gruppen **OR**^{**2**} enthalten.

Neben dem Alkohol, der aus den Silanen freigesetzt wurde, kann die wäßrige Phase noch gegebenenfalls zur Hydrolyse und Kondensation eingesetzten Katalysator enthalten. In der Regel handelt es sich dabei um eine Säure, im einfachsten Fall um Salzsäure, wenn zur Hydrolyse chlorhaltige Silane eingesetzt wurden. Der Wert von **b** beträgt in diesem Fall vorzugsweise durchschnittlich mindestens 0,2, vorzugsweise mindestens durchschnittlich 0,5. Ebenso wie der Alkoholgehalt kann auch der Säuregehalt je nach Harztype stark variieren von 2 bis 20 Gew.-%. Für das bevorzugte Herstellungsverfahren für Harze aus teilalkoxylierten Chlorsilanen liegt die Säurekonzentration zwischen 10 und 20 Gew.-%.

Verantwortlich für die Belegungen von Apparateteilen ist, wenn nicht nach dem erfindungsgemäßen Verfahren gearbeitet wird, der Anteil von im Prozeßabwasser enthaltenen Siliconharz. Dieses liegt in Wasser vor z.B. in Form von emulgierten Tröpfchen oder als dispergierte, gelartige, voluminöse, schmierige Masse. Der Anteil ist wiederum abhängig von der Harztype. Generell steigt die Gelmenge mit dem Anteil von tri- oder tetrafunktionellen Silanen. Im Extremfall kann die Gelmenge bis zu 5 Vol.-% vom Prozeßabwasser betragen.

Wenn die Harzherstellung in Anwesenheit von Lösungsmitteln erfolgt, so finden sich Spuren davon ebenfalls in der Wasserphase und im Gel. Eingesetzt werden können Lösungsmittel, die mit Wasser große Mischungslücken bei Normalbedingungen aufweisen, z.B. aliphatische und aromatische Kohlenwasserstoffe im Siedebereich bis 250 °C oder Ester und Ketone mit mehr als 3 C-Atomen. Bevorzugt eingesetzt werden aromatische Kohlenwasserstoffe, besonders bevorzugt Toluol und Xylol.

Weitere im Prozeßabwasser vorhandene Komponenten können sein Vergällungsmittel aus dem Alkohol, welcher zur Umsetzung der Kohlenwasserstoffchlorsilane zu den Silanen der allgemeinen Formel I eingesetzt worden ist; Verunreinigungen, die über die Ausgangsstoffe eingeschleppt werden, z.B. gesättigte oder ungesättigte Kohlenwasserstoffe aus den Kohlenwasserstoffchlorsilanen; und Alkylhalogenide, die sich während der Alkoxylierung oder Hydrolyse aus Olefinen durch Addition oder aus den Alkoholen durch Substitutionsreaktionen mit Halogenwasserstoffsäuren bilden können. Der Anteil dieser weiteren im Abwasser vorhandenen Komponenten beträgt vorzugsweise höchstens 1 Gew.-%.

Im ersten Schritt wird zu dem oben beschriebenen Prozeßabwasser Lauge dosiert. Es können sowohl organische, wie auch anorganische Basen in reiner Form oder in Form einer wäßrigen Lösung eingesetzt werden. Bevorzugt werden wäßrige Lösungen von Alkalihydroxiden, insbesondere Natronlauge eingesetzt. Die Konzentration an Alkalihydroxid beträgt vorzugsweise 20 - 50 Gew.-%.

Der Zusatz von Lauge erfolgt vorzugsweise bei Temperaturen von 0°C bis 40°C, insbesondere 10°C bis 30°C und vorzugsweise bei 0,05 MPa bis 1 MPa und kontinuierlich. Aber auch abweichende Temperaturen und Drücke oder diskontinuierliche Zugabe sind nicht ausgeschlossen. Die Dosierung erfolgt so, daß der pH-Wert der Mischung mindestens 8 ist, bevorzugt 11 bis 13.

Der Zusatz von Lauge bewirkt äußerst rasche Kondensation der Harz- und Geltröpfchen zu unlöslicher Organokieselsäure. Diese Organokieselsäure ist sehr restgruppenarm und besitzt nur eine geringe Oberflächenenergie. Sie legt sich daher auf Oberflächen nicht mehr an und kann in Form feiner Partikel mühelos filtriert werden.

Im Gegensatz dazu verläuft die Kondensation im Sauren langsamer und es bilden sich restgruppenreichere Harze, die langsam an allen angebotenen Oberflächen aufwachsen und Beläge bilden, so daß periodisch abgereinigt werden muß. Daher muß bei saurer Aufarbeitung auch dafür gesorgt werden, daß die Gelanteile im Prozeßabwasser minimiert sind, was z.B. durch zusätzliche Abscheideeinrichtungen erfolgen kann. Dieser zusätzliche Aufwand fällt bei einer alkalischen Aufarbeitung weg.

Der zweite wichtige Effekt, den die Lauge ausübt, ist die Minimierung der Alkoholverluste über eine Substitutionsreaktion mit den bevorzugt eingesetzten Halogenwasserstoffsäuren zu Alkylhalogeniden, die ihrerseits ein bedeutendes Emissionsproblem darstellen.

Der zweite Schritt ist die Dampfstrippung. Dazu wird das alkalische Gemisch aus dem ersten Schritt in eine Kolonne eingespeist. Günstigerweise handelt es sich um eine Blasensäule ohne zusätzliche Einbauten, um eine Verlegung aufgrund von Harzpartikeln zu vermeiden. Gleichzeitig wird Dampf eingespeist. Die Dosierung kann sowohl im Gleichstrom als auch im Gegenstrom erfolgen. Bevorzugt ist die Dosierung im Gegenstrom mit Dampfeinspeisung im Sumpf und Dosierung des alkalischen Gemisches in der oberen Hälfte der Kolonne. Das Verhältnis von Dampf und alkalischem Gemisch ist abhängig vom Alkoholgehalt des Gemisches und vom Energieinhalt des eingesetzten Dampfes und kann deshalb nur exemplarisch angegeben werden (siehe Beispiele).

Das im zweiten Schritt verdampfte Gemisch aus Alkohol und Wasser wird vorzugsweise kondensiert und ist neutral sowie frei von testen und geligen Anteilen. Lediglich im Prozeßabwasser enthaltene Lösungsmittel können noch vorhanden sein. Das Verhältnis Alkohol:Wasser kann je nach Bedingungen in der Dampfstrippung stark variieren. Günstigerweise liegt es allerdings bei mindestens 1:2, bevorzugt mindestens 1:1.

Der Restalkoholgehalt des aus der Blasensäule abgezogenen Inhalts liegt üblicherweise unter 1 Gew.-%.

Das im Verfahren abgetrennte Alkohol-Wasser-Gemisch kann bei geeigneter Prozeßführung direkt bei der Harzherstellung eingesetzt werden.

Vorzugsweise wird der abgetrennte Alkohol von restlichem Wasser ganz oder teilweise befreit. Dabei wird vorzugsweise, z.B. über eine Destillation oder Trennung über ein Membranverfahren, der Alkohol aufkonzentriert auf 90 - 100 Gew.-% Gehalt und dann bei der Harzherstellung wieder eingesetzt.

In den nachfolgenden Beispielen sind, falls jeweils nicht anders angegeben,
a) alle Mengenangaben auf das Gewicht bezogen;
b) alle Drücke 0,10 MPa (abs.);
c) alle Temperaturen 20°C .

### Beispiele:

Für die Beispiele wurde eingesetzt ein Prozeßabwasser aus der Methylsiliconharzproduktion mit folgender Zusammensetzung:

| Komponente | Anteil | Mengenstrom |
|---|---|---|
| | [Gew.-%] | [kg/h] |
| Wasser | 68,3 | 2,05 |
| Ethanol | 21 | 0,63 |
| HCl | 10 | 0,3 |
| Toluol | 0,1 | 0 |
| Harz/Gel-Anteil | 0,5 | 0,02 |
| Ethylchlorid | 0,1 | 0 |

### Beispiel 1:

Das Prozeßabwasser wurde mit wäßriger NaOH (25 Gew.-%) auf pH 12 eingestellt. Eine Blasensäule (⌀ 100 mm, Länge 2000 mm, Duran-Glas) wurde zu 60 % mit dem Prozeßabwasser gefüllt. Das Prozeßabwasser konnte kontinuierlich mittels einer Membranpumpe in Höhe des Flüssigkeitsspiegels dosiert werden. Im Sumpf konnte ebenfalls kontinuierlich mittels einer Membranpumpe das von Alkohol abgereicherte Prozeßabwasser abgezogen werden. 100 mm über dem Sumpfabzug wurde über eine Düse Dampf mit einer Temperatur von 160 °C eingespeist. Das Verhältnis Dampf: Prozeßabwasser betrug 0,9. Die Dosierung des Prozeßabwassers betrug 3 kg/h.

Das Destillat bestand aus:

| Komponente | Anteil | Mengenstrom |
|---|---|---|
| | [Gew.-%] | [kg/h] |
| Wasser | 29,3 | 0,22 |
| Ethanol | 69,9 | 0,53 |
| Toluol | 0,4 | 0 |
| Ethylchlorid | 0,4 | 0 |

Der Sumpfabzug enthielt feine Methylkieselsäurepartikel und 0,8 Gew.-% Ethanol. Die Glaskolonne war nach 100 h ohne Belag.

Es wurde nur die bereits vorhandene Ethylchloridmenge gefunden, es fand keine Neubildung statt.

### Beispiel 2 (Vergleichsbeispiel):

Beispiel 1 wurde wiederholt, allerdings wurde das Prozeßabwasser nicht vorbehandelt. Bereits nach wenigen Stunden begann sich auf dem Glas ein Überzug zu bilden. Bei Versuchsende nach 100 h war die Sumpfabzugsleitung zu fast der Hälfte des Querschnitts zugewachsen und mußte gereinigt werden. Der Sumpfabzug enthielt wenige, unregelmäßig große Methylkieselsäurepartikel aus Abplatzungen von Wandbelägen und 0,9 Gew.-% Ethanol.

Das Destillat bestand aus:

| Komponente | Anteil | Mengenstrom |
|---|---|---|
| | [Gew.-%] | [kg/h] |
| Wasser | 29,3 | 0,22 |
| Ethanol | 69,1 | 0,52 |
| Toluol | 0,4 | 0 |
| Ethylchlorid | 1,2 | 0,01 |

Es fand Neubildung von Ethylchlorid statt.

## Patentansprüche

1. Verfahren zur Aufarbeitung des Alkohol enthaltenden Abwassers, welches bei der Hydrolyse von Silanen der allgemeinen Formel I
R¹ₐSiCl_{b}OR²_{4-a-b} (I),
in der
**R**^{**1**} Wasserstoffatome oder gleiche oder verschiedene einwertige, gegebenenfalls halogensubstituierte, SiC-gebundene C₁-C₁₈-Kohlenwasserstoffreste,
**R**^{**2**} gleiche oder verschiedene einwertige C₁-C₁₀-Kohlenwasserstoffreste,
**a** 0, 1, 2 oder 3, durchschnittlich 0,3 bis 1,9 und
**b** 0, 1, 2, 3 oder 4, durchschnittlich 0,0 bis 3,0
bedeuten, mit der Maßgabe, daß die Summe **a+b** durchschnittlich höchstens 3,5 beträgt,
zur Herstellung von Siliconharzen anfällt, bei dem in einem ersten Schritt das Abwasser mit Lauge alkalisch gestellt wird und
in einem zweiten Schritt aus der entstandenen alkalischen Lösung oder Suspension der Alkohol durch Einblasen von Wasserdampf entfernt wird.

2. Verfahren nach Anspruch 1, bei dem **R**^{**1**} einen Methyl- oder Phenylrest bedeutet.

3. Verfahren nach Anspruch 1 oder 2, bei dem **R**^{**2**} einen Methyl- oder Ethylrest bedeutet.

4. Verfahren nach einem der Ansprüche 1 bis 3, bei dem der Alkoholgehalt des Alkohol enthaltenden Abwassers der Hydrolyse 5 bis 50 Gew.-% beträgt.

5. Verfahren nach einem der Ansprüche 1 bis 4, bei dem im ersten Schritt als Lauge eine wäßrige Lösung von Alkalihydroxid eingesetzt wird.

6. Verfahren nach einem der Ansprüche 1 bis 5, bei dem im ersten Schritt mit der Lauge ein pH-Wert des Abwassers von mindestens 8 eingestellt wird.

## Claims

1. Process for working up the alcohol-containing wastewater which is obtained in the hydrolysis of silanes of the general formula I
R¹ₐSiCl_{b}OR²_{4-a-b} (I)
, where
R¹ are hydrogen atoms or identical or different monovalent, unsubstituted or halogen-substituted, SiC-bonded C₁-C₁₈-hydrocarbon radicals,
R² are identical or different monovalent C₁-C₁₀-hydrocarbon radicals,
a is 0, 1, 2 or 3, on average from 0.3 to 1.9, and
b is 0, 1, 2, 3 or 4, on average from 0.0 to 3.0,
with the proviso that the sum a + b is on average at most 3.5,
for the preparation of silicone resins, wherein, in a first step, the wastewater is made alkaline by addition of alkali and,
in a second step, the alcohol is removed from the resulting alkaline solution or suspension by injection of steam.

2. Process according to Claim 1, wherein R¹ is a methyl or phenyl radical.

3. Process according to Claim 1 or 2, wherein R² is a methyl or ethyl radical.

4. Process according to any of Claims 1 to 3, wherein the alcohol content of the alcohol-containing wastewater from the hydrolysis is from 5 to 50% by weight.

5. Process according to any of Claims 1 to 4, wherein, in the first step, the alkali used is an aqueous solution of alkali metal hydroxide.

6. Process according to any of Claims 1 to 5, wherein, in the first step, the pH of the wastewater is adjusted to at least 8 by addition of the alkali.

## Revendications

1. Procédé de traitement des eaux usées contenant de l'alcool qui sont obtenues lors de l'hydrolyse de silanes de formule générale I
R¹aSiCl_{b}OR²4-a-b (I),
dans laquelle
R¹ représente des atomes d'hydrogène ou des radicaux hydrocarbonés en C₁-C₁₈ monovalents, identiques ou différents, liés à SiC et éventuellement halogénosubstitués,
R² représente des radicaux hydrocarbonés en C₁-C₁₀ monovalents, identiques ou différents,
a vaut 0, 1, 2 ou 3, en moyenne de 0,3 à 1,9, et
b vaut 0, 1, 2, 3 ou 4, en moyenne de 0,0 à 3,0,
à condition que la somme a + b vaille en moyenne au plus 3,5,
pour la préparation de résines de silicone, dans lequel dans une première étape, les eaux usées sont rendues alcaline à l'aide d'une lessive alcaline et,
dans une deuxième étape, l'alcool est éliminé de la solution ou suspension alcaline résultante par injection de vapeur d'eau.

2. Procédé selon la revendication 1, dans lequel R¹ représente un radical méthyle ou phényle.

3. Procédé selon la revendication 1 ou 2, dans lequel R² représente un radical méthyle ou éthyle.

4. Procédé selon l'une quelconque des revendications 1 à 3, dans lequel la teneur en alcool des eaux usées de l'hydrolyse, contenant de l'alcool, est de 5 à 50% en poids.

5. Procédé selon l'une quelconque des revendications 1 à 4, dans lequel, dans la première étape, on utilise une solution aqueuse d'hydroxyde alcalin en tant que lessive alcaline.

6. Procédé selon l'une quelconque des revendications 1 à 5, dans lequel, dans la première étape, le pH des eaux usées est ajusté à au moins 8 à l'aide de la lessive alcaline.
